# FASCICULE DE BREVET EUROPEEN

(11) **EP 1 165 559 B1**
(45) Date de publication et mention de la délivrance du brevet: **19.02.2003**
(21) Numéro de dépôt: 00911011.5
(22) Date de dépôt: 21.03.2000
(51) Int. Cl.: C07D 471/08, A61K 31/551, A61P 25/00

(54) **DERIVES DE 1,4-DIAZABICYCLO[3.2.2]NONANE-4-CARBOXYLATES ET CARBOXAMIDES, LEUR PREPARATION ET LEUR APPLICATION EN THERAPEUTIQUE**
1,4-DIAZABICYCLO[3.2.2]NONAN-4-CARBOXYLAT UND CARBOXAMIDDERIVATE, IHRE HERSTELLUNG UND THERAPEUTISCHE VERWENDUNG
1,4-DIAZABICYCLO[3.2.2]NONANE-4-CARBOXYLATE AND CARBOXAMIDE DERIVATIVES, PRODUCTION AND USE THEREOF IN THERAPEUTICS

(30) Priorité: 30.03.1999 FR 9903934
(43) Date de publication de la demande: 02.01.2002
(73) Titulaire: SANOFI-SYNTHELABO, 75013 Paris (FR)
(72) Inventeur: GALLET, Thierry, F-91120 Palaiseau (FR); JEGHAM, Samir, F-34980 Montferrier sur Lez (FR); LARDENOIS, Patrick, F-92340 Bourg-La-Reine (FR); LOCHEAD, Alistair, F-94220 Charenton (FR); NEDELEC, Alain, F-92700 Colombes (FR)
(74) Mandataire: Ludwig, Jacques
(86) Numéro de dépôt international: FR0000697
(87) Numéro de publication internationale: WO00058311

(56) Documents cités:
- EP-A- 0 235 878
- B.R. DE COSTA ET AL.: "Synthesis and evaluation of comformationally restricted N-[2-(3,4-dichlorophenyl)ethyl]-N-methyl-2 -(1-pyrrolidinyl)ethylamines at sigma receptors. 2. Piperazines, bicyclic amines, bridged bicyclic amines, and miscellaneous compounds." JOURNAL OF MEDICINAL CHEMISTRY., vol. 36, no. 16, 1993, pages 2311-2320, XP002125229 AMERICAN CHEMICAL SOCIETY. WASHINGTON., US ISSN: 0022-2623 cité dans la demande

## Description

La présente invention a pour objet des composés de formule générale (I) 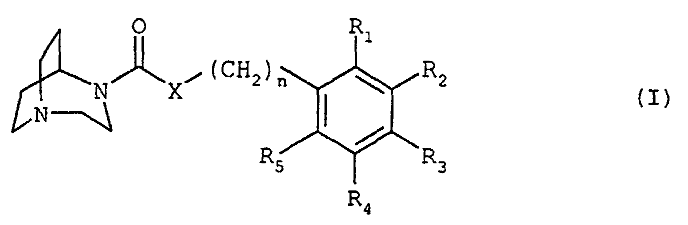 dans laquelle
X représente un atome d'oxygène ou un groupe de formule NZ dans laquelle Z représente un atome d'hydrogène ou un groupe (C₁-C₆) alkyle,
n représente le nombre 0, 1 ou 2, et
R₁, R₂, R₃, R₄ et R₅ représentent chacun, indépendamment l'un de l'autre, un atome d'hydrogène ou d'halogène ou un groupe trifluorométhyle, trifluorométhoxy, cyano, hydroxy, (C₁-C₆)alkyle, (C₁-C₆)alcoxy, phénoxy ou phényle éventuellement substitué par un atome d'halogène ou un groupe trifluorométhyle, cyano, hydroxy, (C₁-C₆)alkyle ou (C₁-C₆)alcoxy, ou bien encore R₂ et R₃ forment ensemble un groupe de formule -OCH₂O- ou -CH₂CH₂CH₂CH₂-.

Les composés de l'invention peuvent exister à l'état de bases ou de sels d'addition à des acides.

Pour préparer les composés de formule générale (I) on peut faire réagir le 1,4-diazabicyclo[3.2.2]nonane avec un composé de formule générale (II) 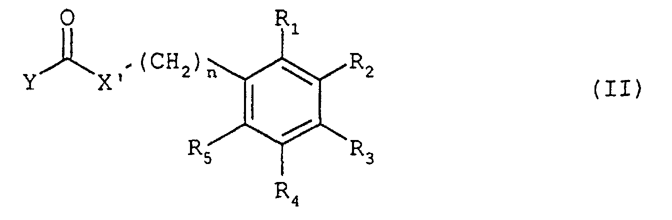 dans laquelle n, R₁, R₂, R₃, R₄ et R₅ sont tels que définis ci-dessus, X' représente un atome d'oxygène ou un groupe de formule N-alkyle et Y représente un atome d'halogène, en présence d'une base telle que la triéthylamine ou la pyridine.
Pour préparer les composés de formule générale (I) dans laquelle X représente un groupe NH, on peut faire réagir le 1,4-diazabicyclo[3.2.2]nonane avec un isocyanate de formule générale (III) 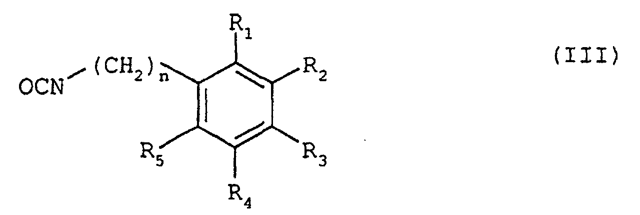 dans laquelle n, R₁, R₂, R₃, R₄ et R₅ sont tels que définis ci-dessus, dans des conditions identiques à celles décrites ci-dessus.

Le 1,4-diazabicyclo[3.2.2]nonane est décrit dans *J. Med. Chem.* (1993) **36** 2311-2320.
Les composés de formules générales (II) et (III) sont disponibles dans le commerce, ou peuvent être préparés selon toutes méthodes connues.

Les exemples qui vont suivre illustrent la préparation de quelques composés de l'invention. Les microanalyses élémentaires, et les spectres I.R. et R.M.N. confirment les structures des composés obtenus. Les numéros indiqués entre parenthèses dans les titres des exemples correspondent à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°2).

### 1,4-Diazabicyclo[3.2.2]nonane-4-carboxylate de 4-bromophényle.

Dans un ballon tricol de 50 ml, on introduit 0,379 g (3,0 mmoles) de 1,4-diazabicyclo[3.2.2]nonane et 0,84 ml (6,0 mmoles) de triéthylamine dans 5 ml de dichlorométhane, on refroidit le mélange à 0°C, on ajoute, goutte à goutte, 0,730 mg (3,1 mmoles) de chloroformiate de 4-bromophényle en solution dans 3 ml de dichlorométhane et on maintient l'agitation à 0°C pendant 10 min.
On lave le milieu réactionnel à l'eau, on lave la phase aqueuse deux fois au dichlorométhane, on lave les phases organiques réunies avec une solution aqueuse saturée de chlorure de sodium, on sèche et on évapore le solvant sous pression réduite. On purifie le résidu obtenu par chromatographie sur gel de silice en éluant avec un mélange 95/5/0,5 de chloroforme, méthanol et ammoniaque. On obtient un produit brut que l'on triture dans l'éther diisopro_ pylique.
On obtient ainsi 0,77 g de produit pur sous forme de solide blanc.
Point de fusion : 115-116°C.

### Exemple 2 (Composé N°8)

### Bromhydrate de N-phényl-1,4-diazabicyclo[3.2.2]nonane-4-carboxamide (1:1).

Dans un ballon tricol de 25 ml on introduit 0,378 g (3,0 mmoles) de 1,4-diazabicyclo[3.2.2]nonane en solution dans 10 ml d'acétonitrile, on ajoute à 3°C une solution de 0,358 g (3,0 mmoles) d'isocyanatobenzène dans 2 ml d'acétonitrile et on agite le milieu réactionnel pendant 10 min à température ambiante.
On évapore le solvant sous pression réduite pour obtenir un solide que l'on dissout dans 30 ml d'éthanol et que l'on traite avec 0,53 ml d'une solution d'acide bromhydrique 5,7 M dans l'acide acétique à 50°C. On filtre le précipité qui se forme et on le lave deux fois avec de l'éthanol.
On obtient ainsi 0,649 g de produit sous forme de solide blanc.
Point de fusion : 229-231°C.

### Exemple 3 (Composé N°10).

### Bromhydrate de N-méthyl-N-phényl-1,4-diazabicyclo[3.2.2]nonane-4-carboxamide (1:1).

Dans un ballon tricol de 25 ml on introduit 0,69 ml (1,31 mmole) d'une solution de phosgène à 20% dans le toluène diluée par addition de 4 ml de toluène et on refroidit la solution à 0°C. On ajoute une solution de 0,127 g (1,12 mmole) de *N*-méthylaniline et 0,11 ml de pyridine dans 4 ml de toluène pendant 10 min et on laisse sous agitation magnétique pendant 30 min à 0°C.
On ajoute 10 ml d'eau glacée et on séparé la phase organique. Dans un ballon tricol de 25 ml, on verse cette solution sur une suspension contenant 0,15 g (1,12 mmole) de 1,4-diazabicyclo[3.2.2]nonane dans 0,11 ml de pyridine et on agite le mélange pendant 30 min.
On ajoute 10 ml de chloroforme, on lave la solution obtenue avec 15 ml d'une solution aqueuse d'hydroxyde de sodium 1 M, on évapore le solvant et on purifie le résidu par chromatographie sur gel de silice en éluant avec un mélange 95/5/0,5 de chloroforme, méthanol et diéthylamine.
On obtient 0,31 g de produit que l'on reprend dans 5ml d'éthanol, on ajoute 0,109 ml d'une solution aqueuse d'acide bromhydrique, on dilue le milieu par addition de 5 ml d'éther diisopropylique et on recueille le précipité par filtration.
On obtient ainsi 0,387 g de produit sous forme de solide blanc.
Point de fusion : 292-293°C.

### Exemple 4 (Composé N°11).

### Bromhydrate de 1,4-diazabicyclo[3.2.2]nonane-4-carboxylate de [1,1'-biphényl-4-yle] (1:1).

### 4.1. Chloroformiate de [1,1'-biphényl-4-yle].

Préparation selon la méthode décrite dans *Bull. Soc. Chim.* Fr. (1967).
Dans un ballon tricol de 50 ml on introduit 2,00 g (11,75 mmoles) de [1,1'-biphényl]-4-ol en suspension dans 50 ml de dichlorométhane, on ajoute par portions 0,47 g (11,75 mmoles) d'hydrure de sodium à 60% dans l'huile minérale, et on évapore le solvant sous pression réduite. On obtient un solide blanc que l'on ajoute en 1 h à 6,84 ml (12,92 mmoles) d'une solution de phosgène à 20% dans le toluène à 30°C et on laisse en contact pendant 3 h.
On évapore le solvant sous pression réduite, on triture le résidu dans de l'éther de pétrole, on filtre pour retirer les minéraux et on évapore le solvant sous pression réduite.
On obtient ainsi 0,89 g de produit brut.
Point de fusion : 36 °C.

### 4.2. Bromhydrate de 1,4-diazabicyclo[3.2.2]nonane-4-carboxylate de [1,1'-biphényl-4-yle] (1:1).

Dans un ballon tricol de 50 ml on introduit 0,15 g (1,19 mmole) de 1,4-diazabicyclo[3.2.2]nonane et 0,33 ml (2,38 mmoles) de triéthylamine en solution dans 10 ml de chloroforme, on refroidit le mélange à 0°C, on ajoute ensuite, en 10 min, le chloroformiate obtenu précédemment en solution dans 10 ml de chloroforme. On agite à 0°C pendant 10 min avant de laisser la température monter jusqu'a l'ambiante et on laisse à température ambiante pendant 18 h.
On ajoute 15 ml de soude 1 M et on extrait au chloroforme. On évapore le solvant sous pression réduite et on purifie le résidu obtenu par chromatographie sur gel de silice en éluant avec un mélange 98/2/0,2 puis 96/4/0,4 de chloroforme, methanol et diéthylamine.
On obtient 0,31 g de produit qu'on dissout dans 5 ml d'éthanol, on traite la solution avec 0,109 ml (0,96 mmole) d'une solution aqueuse d'acide bromhydrique, on ajoute 5 ml d'éther diisopropylique et on filtre le précipité.
On obtient ainsi 0,387 g de produit sous forme de solide blanc.
Point de fusion : 292-293 °C.

Le tableau qui suit illustre les structures chimiques et les propriétés physiques de quelques composés de l'invention.

Dans la colonne "Sel", "-" désigne un composé à l'état de base, "HBr" désigne un bromhydrate et "ox" désigne un oxalate, ou éthanedioate ; le rapport molaire acide:base est indiqué en regard.

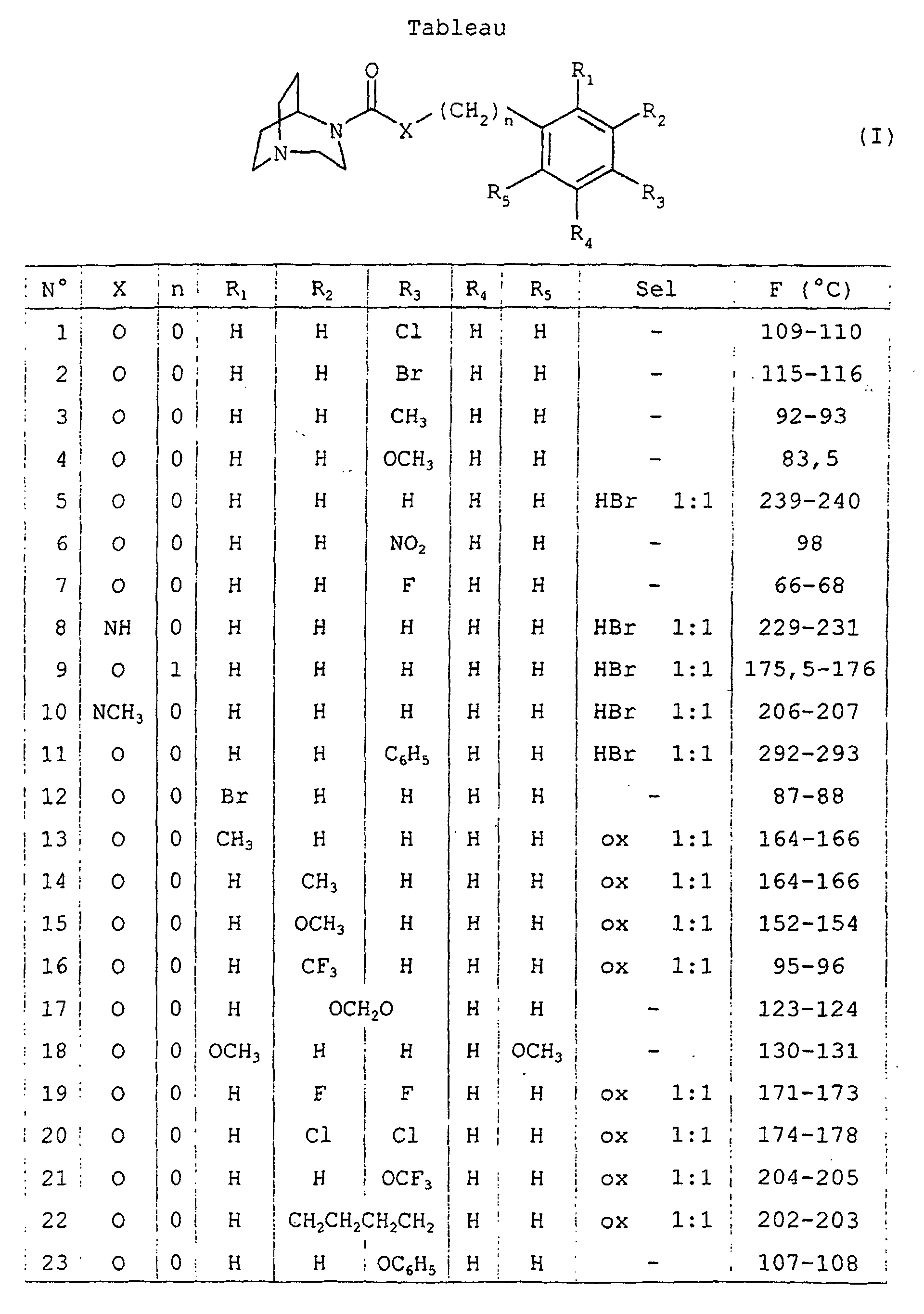

Les composés de l'invention ont fait l'objet d'essais qui ont mis en évidence leurs propriétés thérapeutiques.

Les composés de l'invention ont aussi été étudiés quant à leur affinité vis-à-vis des récepteurs nicotiniques contenant la sous-unité α7, selon les méthodes décrites par Marks et Collins, *J.Pharmacol.Exp.Ther.* (1982) **22** 554 et Marks et al., *Mol. Pharmacol.* (1986) **30** 427.
On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 g pendant 10 min. On élimine le culot, et on centrifuge le surnageant à 8000 g pendant 20 min à 4°C. On récupère le culot et on l'homogénéise à l'aide d'un broyeur Polytron™ dans 15 volumes d'eau bidistillée à 4°C, puis on le centifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.
Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis le membranes sont incubées pendant 60 min à 37°C, à l'obscurité, en présence de 50 µl de [³H]α-bungarotoxine 1 nM dans un volume final de 250 µl de tampon HEPES 20 mM à 0,05% de polyéthylèneimine. On arrête la réaction par filtration sur des filtres Whatman GF/C™ préalablement traités pendant 3 heures avec de la polyéthylèneimine à 0,5%. On rince les filtres avec deux fois 5 ml de tampon à 4°C, et on mesure la radioactivité retenue sur chaque filtre par scintigraphie liquide. On détermine la liaison non spécifique en présence de α-bungarotoxine à 1 µM final ; la liaison non spécifique représente environ 60% de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.
Les CI₅₀ des composés de l'invention les plus affins se situent entre 0,04 et 0,5 µM.

Les résultats des essais qui précèdent montrent que les composés de l'invention sont des ligands pour les sous-unités α₇ du récepteur nicotinique.

Ces résultats suggèrent l'utilisation des composés dans le traitement ou la prévention des désordres liés à un dysfonctionnement des récepteurs nicotiniques, notamment au niveau du système nerveux central ou du système gastro-intestinal.

Au niveau du système nerveux central ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires(multi-infarct dementia, MID).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des accidents vasculaires cérébraux et des épisodes hypoxiques cérébraux.
Ils peuvent être utilisés dans les cas de pathologies psychiatriques : schizophrénie, dépression, anxiété, attaques de panique, comportements compulsifs et obsessionnels.
Ils peuvent prévenir les symptômes dus au sevrage au tabac, à l'alcool, aux différentes substances induisant une dépendance, telles que cocaïne, LSD, cannabis, benzodiazépines.

Au niveau du système gastro-intestinal les composés de l'invention pourraient être utiles dans le traitement de la maladie de Crohn, de la colite ulcéreuse, du syndrome du côlon irritable et de l'obésité.

A cet effet les composés de l'invention peuvent être présentés sous toutes formes de compositions appropriées à l'administration entérale, parentérale ou transdermique, telles que comprimés, dragées, gélules, capsules, suspensions ou solutions buvables ou injectables telles que sirops ou ampoules, timbres transdermiques ("patch"), etc, associés à des excipients convenables, et dosés pour permettre une administration journalière de 0,01 à 20 mg/kg.

## Revendications

1. Composé répondant à la formule générale (I) 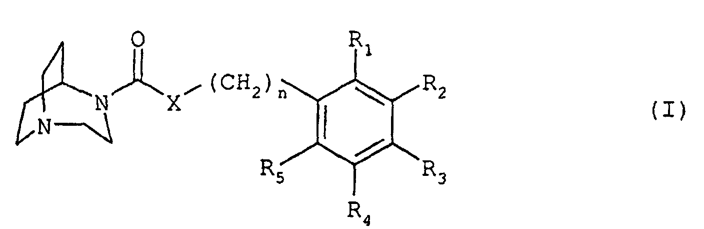 dans laquelle
X représente un atome d'oxygène ou un groupe de formule NZ dans laquelle Z représente un atome d'hydrogène ou un groupe (C₁-C₆)alkyle,
n représente le nombre 0, 1 ou 2, et
R₁, R₂, R₃, R₄ et R₅ représentent chacun, indépendamment l'un de l'autre, un atome d'hydrogène ou d'halogène ou un groupe trifluorométhyle, trifluorométhoxy, cyano, hydroxy, (C₁-C₆)alkyle, (C₁-C₆)alcoxy, phénoxy ou phényle éventuellement substitué par un atome d'halogène ou un groupe trifluorométhyle, cyano, hydroxy, (C₁-C₆)alkyle ou (C₁-C₆)alcoxy, ou bien encore R₂ et R₃ forment ensemble un groupe de formule -OCH₂O- ou -CH₂CH₂CH₂CH₂-,
à l'état de base ou de sel d'addition à un acide.

2. Médicament **caractérisé en ce qu'**il consiste en un composé selon la revendication 1.

3. Composition pharmaceutique **caractérisée en ce qu'**elle contient un composé selon la revendication 1, associé à un excipient.

## Patentansprüche

1. Verbindung der allgemeinen Formel (I) 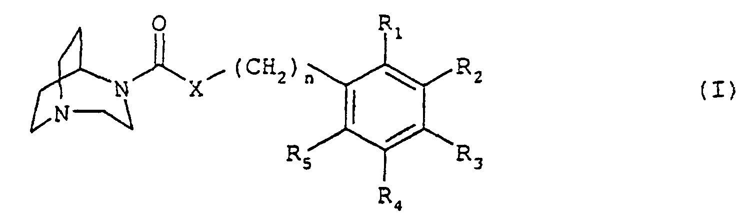 in der
X ein Sauerstoffatom oder eine Gruppe der Formel NZ, in der Z ein Wasserstoffatom oder eine (C₁-C₆)-Alkylgruppe darstellt,
n die Zahl 0, 1 oder 2 und
R₁, R₂, R₃, R₄ und R₅ jeweils unabhängig voneinander Wasserstoffatome, Halogenatome, Trifluormethyl-, Trifluormethoxy-, Cyano-, Hydroxy-, (C₁-C₆)-Alkyl-, (C₁-C₆)-Alkoxy-, Phenoxy- oder Phenylgruppen, gegebenenfalls substituiert durch ein Halogenatom oder eine Trifluormethyl-, Cyano-, Hydroxy-, (C₁-C₆)-Alkyl- oder (C₁-C₆)-Alkoxygruppe bedeuten, oder R₂ und R₃ gemeinsam eine Gruppe der Formel -OCH₂O- oder -CH₂CH₂CH₂CH₂- bilden,
in Form der Base oder eines Säureadditionssalzes.

2. Arzneimittel, **dadurch gekennzeichnet, daß** es aus einer Verbindung nach Anspruch 1 besteht.

3. Pharmazeutische Zubereitung, **dadurch gekennzeichnet, daß** sie eine Verbindung nach Anspruch 1 in Kombination mit einem Trägermaterial enthält.

## Claims

1. Compound corresponding to the general formula (I) 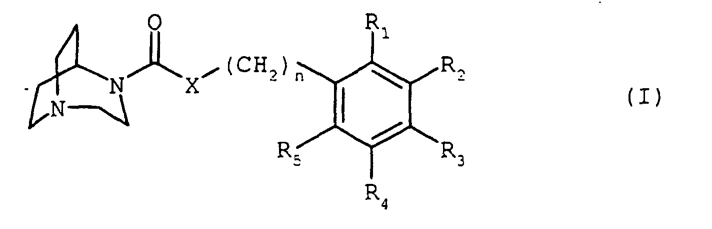 in which
X represents an oxygen atom or a group of formula NZ in which Z represents a hydrogen atom or a (C₁-C₆)alkyl group,
n represents the number 0, 1 or 2, and
R₁, R₂, R₃, R₄ and R₅ each represent, independently of each other, a hydrogen or halogen atom or a trifluoromethyl, trifluoromethoxy, cyano, hydroxyl, (C₁-C₆)alkyl, (C₁-C₆)alkoxy, phenoxy or phenyl group optionally substituted with a halogen atom or a trifluoromethyl, cyano, hydroxyl, (C₁-C₆)alkyl or (C₁-C₆)alkoxy group, or alternatively R₂ and R₃ together form a group of formula -OCH₂O- or -CH₂CH₂CH₂CH₂-,
in the form of a base or of an addition salt with an acid.

2. Medicament, **characterized in that** it consists of a compound according to Claim 1.

3. Pharmaceutical composition, **characterized in that** it contains a compound according to Claim 1, combined with an excipient.
